# EUROPEAN PATENT APPLICATION

(11) **EP 2 455 466 A1**
(43) Date of publication of application: **23.05.2012**
(21) Application number: 10799917.9
(22) Date of filing: 16.07.2010
(51) Int. Cl.: C12N 15/00, A01K 67/027, A61K 31/713, A61K 48/00, A61P 43/00, C12N 5/10, C12N 15/09

(54) **DNA FRAGMENT AND PHARMACEUTICAL COMPOSITION**

(30) Priority: 16.07.2009 JP 2009167580
(71) Applicant: Immuno-Biological Laboratories Co., Ltd., Fujioka-shi Gunma 375-0005 (JP)
(72) Inventor: SHINDO, Takayuki, Matsumoto-shi Nagano 3900813 (JP); SAKURAI, Takayuki, Matsumoto-shi Nagano 390-0311 (JP)
(74) Representative: Maggs, Michael Norman
(86) International application number: PCT/JP2010/062042
(87) International publication number: WO 2011/007859

(57) **Abstract**

Provided are a DNA fragment, a pharmaceutical composition, and the like, which can simplify the period of creating knockout animals. The DNA fragment includes: a detection sequence that codes for a detection marker other than drug resistance (preferably a visually-detectable marker); a resistance sequence that codes for a resistance marker for a drug that inhibits the proliferation of one or more species of at least prokaryotic organisms; a promoter sequence that is located upstream of the resistance sequence and functions in prokaryotic organisms; and a regulatory sequence that is located upstream of all of the above sequences and, only when inserted into a target region of the genome of at least one species of eukaryotic organism, induces the expression of a sequence located downstream. The detection sequence and the resistance sequence are arranged so as to be capable of action.

## Description

### TECHNICAL FIELD

The present invention relates to a DNA fragment, a vector, a host cell, a method for manufacturing a non-human knockout animal, a method for screening a candidate compound for a therapeutic agent, and a pharmaceutical composition.

### BACKGROUND ART

The manufacturing of a knockout animal needs a targeting gene vector. A conventional targeting gene vector usually has a sequence in which a base sequence constituting a gene to be knocked-out is partially mutated, and sequences constituting a neomycin resistant gene and a thymidine kinase gene respectively, and these genes are forcedly expressed by a strong promoter (for example, a PGK promoter).

When such a targeting gene vector is introduced into an embryonic stem cell derived from a desired animal and a DNA derived from the vector is inserted into the embryonic stem cell genome, the DNA is induced into the promoter to thereby cause a drug marker to be expressed. This ensures that the modified embryonic stem cell with the genome into which the DNA derived from the vector is inserted is viable in the presence of drugs and can be screened. Next, a Guncyclover is given to the screened cell group to eliminate cell lines into which DNAs are randomly inserted (see, for example, Non-patent Literature 1).

The screened modified embryonic stem cell is infused into an animal embryo and transplanted in the uteruses of recipient females to thereby obtain chimera animals. Each obtained chimera animal is crossed with a wild type animal to select a germline chimera animal based on whether or not the descendant exhibits a phenotype (for example, a coat color) derived from the modified embryonic stem cell. The mating of such germline chimera animals among them results in the creation of a homozygous knockout animal.
Non-Patent Document 1: Circulation 104:1964-71,2001

### DISCLOSURE OF THE INVENTION

### Problems to be Solved by the Invention

In the aforementioned targeting gene vector, however, there exists about 90% of cells other than fine cells with DNAs inserted into desired sites thereof even after the cell lines into which DNAs are randomly inserted are deleted when selecting modified embryonic stem cells. It is therefore necessary to further screen cell lines with DNAs inserted into desired sites thereof by a method, for example, the Southern analysis.

Also, when screening a germline chimera animal, it is necessary to bleed the descendant in readiness for considerable period of time until it came possible to judge whether or not the descendant exhibits a phenotype derived from the modified embryonic stem cell after birth of the descendant. When, for example, mice are used as the animal, many germline chimera animals are screened based on the coat color of the descendant. However, about one week is required to grow hair to the extent necessary to judge the color.

A multistage is required to create a knockout animal, which means that a long period of time is spent in various stages. As a result, an enormous amount of costs are required to create a knockout animal.

The present invention has been made in view of the above situation, and it is an object of the present invention to provide a DNA fragment, a vector, a host cell, a method for manufacturing a gene modified embryonic stem cell or a pluripotent cell, a method for manufacturing a germline chimera animal, and a method for manufacturing a non-human knockout animal, which can simplify he manufacturing of a knockout animal.

It is another object of the present invention to provide a method for screening a candidate compound for a therapeutic agent for diseases using the relevant non-human knockout animal, and a pharmaceutical composition containing the DNA fragment.

### Means for Solving the Problems

The inventors of the present invention have found that the manufacturing a knockout animal can be outstandingly simplified by combining a vector having a detection sequence that codes for a detection marker other than a drug resistance marker, to complete the present invention. Specifically, the followings are provided by the present invention.
(1) A DNA fragment comprising a detection sequence that codes for a detection marker other than a drug resistance marker, a resistance marker that codes for a resistance marker for a drug that inhibits the proliferation of one or more species of at least prokaryotic organisms, a prokaryotic promoter sequence that is located upstream of the resistance sequence and functions in the prokaryotic organisms, and a regulatory sequence that is located the most upstream of the sequences and induces the expression of the sequences located downstream thereof only when it is inserted into a target region of the genome of at least one species of eukaryotic organism, wherein;
      the detection sequence and the resistance sequence are operably arranged.
   (2) The DNA fragment according to the above (1), wherein the regulatory sequence is a sequence constituting one or more types selected from the group consisting of a sequence internal ribosome entry site and a splicing acceptor.
   (3) A DNA fragment comprising a detection sequence that codes for a detection marker which is visually detectable, a resistance sequence that codes for a resistance marker for a drug that inhibits the proliferation of one or more species of at least prokaryotic organisms, a prokaryotic promoter sequence that is located upstream of the resistance sequence and functions in the prokaryotic organisms, and a eukaryotic promoter sequence that is located the most upstream of the sequences and constitutively functions by at least one species of eukaryotic organisms, wherein;
      the detection sequence and the resistance sequence are operably arranged.
   (4) The DNA fragment according to any one of the above (1) to (3), wherein the detection marker is a visually detectable one.
   (5) The DNA fragment according to any one of the above (1) to (4), wherein the resistance marker imparts resistance to drugs that inhibit the proliferation of eukaryotic organisms.
   (6) A vector comprising the sequence of the DNA fragment according to any one of the above (1) to (5).
   (7) The vector according to the above (6), the vector further comprising a complementary sequence of a targeting sequence constituting part of an animal genome.
   (8) A host cell comprising the vector according to the above (6) or (7) introduced thereinto.
   (9) A method for manufacturing a gene modified embryonic stem cell or a gene modified pluripotent cell, the method involving the procedures comprising:
      introducing the vector according to the above (7) into an embryonic stem cell or a pluripotent cell; and
      judging whether or not the detection sequence is expressed in the embryonic stem cell or pluripotent cell after the introduction of the vector to select the embryonic stem cell or pluripotent cell judged to be one in which the expression exists as a gene modified embryonic stem cell or gene modified pluripotent cell.
   (10) A method for manufacturing a germline chimera animal, the method involving the procedures comprising:
      creating a chimera animal by using a gene modified embryonic stem cell or a gene modified pluripotent cell into which the DNA fragment according to any one of the above (1) to (5) is introduced; and
      judging whether or not the detection sequence is expressed in the descendant of the chimera animal to select the chimera animal as a germline chimera animal when the expression is judged to exist in the chimera animal.
   (11) A method for manufacturing a non-human knockout animal, the method involving the procedures comprising:
      introducing the vector according to the above (7) into an embryonic stem cell or a pluripotent cell;
      judging whether or not the detection sequence is expressed in the embryonic stem cell or pluripotent cell after the introduction of the vector to select the embryonic stem cell or pluripotent cell judged to be one in which the expression exists as a gene modified embryonic stem cell or gene modified pluripotent cell;
      creating a germline chimera animal by using the gene modified embryonic stem cell or gene modified pluripotent cell; and
      creating a homozygote by using the germline chimera animal.
   (12) A method for manufacturing a non-human knockout animal, the method involving the procedures comprising:
      creating a chimera animal by using a gene modified embryonic stem cell or a gene modified pluripotent cell into which the DNA fragment according to any one of the above (1) to (5) is introduced; and
      judging whether or not the detection sequence is expressed in the descendant of the chimera animal to select the chimera animal as a germline chimera animal when the expression is judged to exist in the descendant of the chimera animal; and
      creating a homozygote by using the germline chimera animal.
   (13) A method for screening a candidate compound of a therapeutic agent for diseases, the method involving the procedures comprising:
      administering a specimen to a non-human knockout animal in which the causal gene of the diseases is knocked out by introducing the vector according to the above (7); and
      detecting whether or not the disease is improved in the non-human knockout animal.
   (14) A pharmaceutical composition comprising an effective amount of a vector containing the DNA fragment according to any one of the above (1) to (5) and a complementary sequence of a sequence constituting a gene of which repressed expression is effective for the treatment or prevention of diseases, the complementary sequence being located on both sides of the DNA fragment.

### Effects of the Invention

According to the first and second aspects of the present invention, the selection of a gene modified embryonic stem cell or a gene modified pluripotent cell can be accomplished based on the existence of the expression of a resistance sequence which can be determined by life or death of the cell, and on other standard, for example, the expression of a detection sequence which can be visually distinguished, and any of these methods is simple. Also, when screening a germline chimera animal from chimera animals, the presence of the expression of a detection sequence is judged rapidly after birth of a descendant, and if it is determined that a phenotype exists, the chimera animal which is a parent of the descendant may be selected as a germline chimera animal. This reduces the period of time for creating a knockout animal, leading to reduced creating cost.

Also, since a prokaryotic promoter sequence which functions by one or more prokaryotic organisms is located upstream of a resistance sequence, the resistance sequence is expressed when a DNA fragment is introduced into the prokaryotic organisms having a high replication speed to replicate the DNA fragment. Accordingly, cell lines of prokaryotic organisms into which a DNA fragment is introduced can be screened by a drug, so that the DNA fragment can be replicated.

Moreover, the first aspect of the present invention ensures that when screening the gene modified embryonic stem cell or gene modified pluripotent cell, the detection sequence and the resistance sequence are expressed only when the DNA fragment is inserted into a target gene site since the regulatory sequence is located the most upstream of the detection sequence and the resistance sequence. This largely restrains the mixing of cell lines into which DNAs are randomly inserted and therefore, the number of complicated analytical tests such as southern analysis can largely be reduced. Then, the expression of a detection sequence can visually be detected and the expression of a resistance sequence can be determined with the life and death of a cell, making possible to confirm any of these expressions simply. Therefore, the period of time for creating a knockout animal can be shortened, leading to reduce creating cost.

### BRIEF DESCRIPTION OF THE DRAWINGS

FIG. 1 is a schematic view of a DNA fragment according to an embodiment of the present invention.
FIG. 2 is a flowchart showing procedures for screening a host cell according to an embodiment of the present invention.
FIG. 3 is a schematic view of a vector according to an embodiment of the present invention.
FIG. 4 is a view showing the sequence of a regulatory sequence according to an embodiment of the present invention.
FIG. 5 is a schematic view of a vector according to another embodiment of the present invention.
FIG. 6 is a view showing a method for manufacturing the vector shown in FIG. 5.
FIG. 7 is a photograph showing the expression of a detection sequence of the vector shown in FIG. 5.

### PREFERRED MODE FOR CARRYING OUT THE INVENTION

An embodiment of the present invention will be explained with reference to the drawings.

### DNA fragment

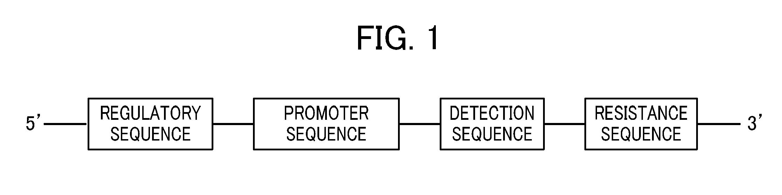

FIG. 1 is a schematic view of a DNA fragment according to an embodiment of the present invention. A DNA fragment according to the present invention includes a detection sequence and a resistance sequence, and a prokaryotic promoter being located upstream of the resistance sequence. Also, a regulatory sequence or an eukaryotic promoter sequence is located the most upstream of these sequences. Each sequence will be explained in detail.

### (Detection sequence)

The detection sequence is a sequence that codes for markers other than a drug resistance marker, for example, visually detectable detection markers. This ensures that when the DNA fragment is introduced into a non-human animal, the detection sequence is expressed in a cell into which the DNA fragment is introduced and this expression is detected, for example, visually, based on a standard different from that of the drug resistance. The cell into which the DNA fragment is introduced can, therefore, be screened easily.

Examples of the visually detectable detection markers generally include, though not particularly limited to, fluorescent proteins, luminescent proteins and proteins developing colors. Examples of the fluorescent proteins include GFP, EGFP, YFP, EYFP, CFP, YCFP, and DsRED. Examples of the luminescent proteins include luciferase, and examples of the proteins developing colors include a-galactosidase and β-galactosidase.

It is noted that if the base length of the detection sequence is longer when the resistance sequence is located downstream of the detection sequence, there is a problem that the resistance sequence is incompletely expressed. In light of this, the detection sequence may be located upstream of the prokaryotic promoter sequence, though it may be located downstream of the prokaryotic promoter sequence which will be explained later as described in this embodiment. The location in this embodiment is, however, preferable in that not only the resistance sequence but also the detection sequence can be utilized for screening in the duplication of a DNA fragment in prokaryotic organisms.

### (Resistance sequence)

The resistance sequence codes for a resistance marker of a drug inhibiting the proliferation of one or more species of at least prokaryotic organisms. When the DNA fragment is introduced into the prokaryotic organisms, the resistance sequence is expressed, as will be mentioned later, therefore, the cell lines of the prokaryotic organisms with the DNA fragment introduced thereinto can be screened easily by a drug.

Examples of such a drug are conventionally known drugs and specific examples of these drugs include kanamycin, ampicillin, chloramphenicol, neomycin, and puromycin. However, the resistance marker is preferably one which imparts resistance to drugs (for example, neomycin and puromycin) which inhibit the proliferation of eukaryotic organisms from the viewpoint of contribution to the screening of cell lines also used when the DNA fragment has been introduced into a non-human animal.

### (Prokaryotic promoter sequence)

The prokaryotic promoter sequence functions in prokaryotic organisms in which the above resistance sequence inhibits the proliferation. For this, when the DNA fragment is introduced into the prokaryotic organisms, the resistance sequence located downstream of the DNA fragment is expressed. Therefore, the cell lines of the prokaryotic organisms with the DNA fragment introduced thereinto can be selected by a drug, so that the DNA fragment can be replicated with ease. Also, a promoter which functions in prokaryotic organisms does not generally function in eukaryotic organisms. For this, the promoter is not a hindrance to the screening of cell lines of eukaryotic organisms with the DNA fragment introduced into a target gene site.

As the prokaryotic promoter sequence, promoters derived from the genomes of prokaryotic organisms can widely be used without any particular limitation, and specific examples of the prokaryotic promoter sequence include an EM7 promoter. When a termination codon exists in the sequence, it should be mutated taking it into account that the sequence is expressed in eukaryotic organisms.

### (Regulatory sequence)

The regulatory sequence induces the expression of a sequence located downstream thereof only when it is inserted into a target site of a genome of eukaryotic organisms. In other words, the transcriptions of the detection sequence and the resistance sequence are not induced when the regulatory sequence is inserted into a site other than the target site. Therefore, only cell lines of eukaryotic organisms in which the DNA fragment is inserted into the target site can be screened by a drug or measures (for example, visual detection) other than drugs.

The regulatory sequence may be a sequence inducing the expression of a sequence located downstream thereof depending on, for example, the translation mechanism of a target gene. Specific examples of the regulatory sequence include an internal ribosome entry site (IRES), a splicing acceptor (SA), and 2A segment (FMDV-2A Segment) derived from a foot-mouth-disease virus (FMDV). IRES is a sequence that initiates cap-independent translation from an internal start site, and SA is a signal sequence of downstream side of an intron necessary for splicing for post-transcriptional control of a gene.

IRES is essential when the DNA fragment is inserted between a promoter driving a target gene of the genome of eukaryotic organisms and exon. On the other hand, when the DNA fragment is inserted between exons of a target gene of a genome of eukaryotic organisms, SA is essential and, in this case, IRES preferably coexists. Therefore, the regulatory sequence is preferably provided with both of IRES and SA from the viewpoint of making it possible to cope with a variety of insertion sites.

When the DNA fragment is utilized for the creation of a gene modified embryonic stem cell or gene modified pluripotent cell, it is preferable to use a DNA fragment including a regulatory sequence if a gene sufficiently expressed in an embryonic stem cell or pluripotent cell is a target. When the DNA fragment is thereby inserted in the sequence of the target gene, the detection sequence and the resistance sequence are expressed by utilizing the expression of the target gene after the vector is introduced into the embryonic stem cell or pluripotent cell, which enables easy screening of a gene modified embryonic stem cell or gene modified pluripotent cell.

### [Eukaryotic promoter sequence]

The eukaryotic promoter sequence constitutively functions by one or more species of eukaryotic organisms and constitutively induces the expression of a sequence located downstream thereof. Namely, the detection sequence and the resistance sequence are resultantly expressed whether or not the insertion site is a target site. As the eukaryotic promoter sequence, conventionally known sequences such as CAG, U6, EF1-α and PGK promoter may be used.

When the DNA fragment is utilized for creating a gene modified embryonic stem cell or gene modified pluripotent cell, it is preferable to use a DNA fragment containing not a regulatory sequence but an eukaryotic promoter sequence if a gene which is insufficiently expressed in an embryonic stem cell or pluripotent cell is a target. This makes it possible to determine easily whether or not the DNA fragment is inserted into the genome of the embryonic stem cell or pluripotent cell. In this case, it is necessary to confirm whether or not the insertion site is located within the sequence of the target gene by Southern blotting or the like.

In the above DNA fragment, the detection sequence and the resistance sequence are operably arranged. Here, "operably arranged" means an arrangement within the reading frame enabling the synthesized protein to produce an intended function.

The DNA fragment may either exist independently or be inserted into an arbitrary vector. Also, the DNA fragment may be amplified using PCR, and may be replicated by introducing it into a host cell to culture the host cell when it is inserted into a vector.

### Vector

The vector according to the present invention is provided with a sequence of the above DNA fragment and may be used for the replication of the DNA fragment through cell culturing and for the creation of a non-human knockout animal. A vector for replication is a vector having a replicator that functions in the host cell and may be one having either high copying ability or low copying ability.

It is preferable to arrange a restriction enzyme on both sides of the DNA fragment so that a complementary sequence can be inserted easily. Specifically, a multi-cloning site is preferably arranged. Also, a sequence such that an m-RNA transcribed from the detection sequence or the resistance sequence is provided with a poly-A tail, is preferably arranged downstream of the DNA fragment.

The targeting vector used to create a non-human knockout animal is provided with a complementary sequence of a targeting sequence constituting part of the animal genome, and this complementary sequence is arranged upstream of the regulatory sequence or eukaryotic promoter sequence and the most downstream of the aforementioned sequence.

The targeting vector is also usually provided with a sequence recognized by a recombinase. When such a targeting vector is introduced into an embryonic stem cell or pluripotent cell, the above sequence is replaced with the target gene DNA by homologous recombination to knockout the target gene.

In this case, the complementary sequence of an animal of which the genomic analysis is finished can be obtained from a known genome DNA library whereas the complementary sequence of an animal of which the genomic analysis is not finished can be isolated from the genome using techniques such as PCR.

In this manner, as the vector of the present invention, a conventionally known vector is selected corresponding to its use and is variously modified and used. For example, a vector having a FRT site recognized by recombinase FLP and a loxP site recognized by recombinase Cre are preferable in that the homologous recombination procedures can be simplified.

### Host cell

The host cell according to the present invention is provided with the aforementioned introduced vector. Such a host cell is a cell derived from prokaryotic organisms that can be used for the replication of a DNA fragment and has high replicability and from a non-human animal which can be used to create a knockout animal.

Examples of the prokaryotic organisms include, though not particularly limited to, Escherichia coli, Streptomyces, Bacillus subtilis, Streptococcus, and Staphylococcus. A vector is introduced into a cell group of these prokaryotic organisms and the cell group is placed under a drug circumstance. The cells into which the vector is introduced express a resistance marker to live under a drug circumstance, and can therefore be easily screened and established as cell lines. These cell lines are cultured to create a vector through refining the proliferated cells to obtain a large amount of vectors. In this case, specific procedures such as the introduction of the vector, isolation of the host cell lines and creation of the vectors may be carried out according to normal methods.

Examples of the non-human animal include, though not particularly limited to, mice, rats, rabbits, pigs, goats, and cows.

### [Method for manufacturing a gene modified embryonic stem cell or a gene modified pluripotent cell]

A method for manufacturing a gene modified embryonic stem cell or a gene modified pluripotent cell which is an example of the host cell of these non-human animals involves the procedures comprising: introducing the aforementioned vector into an embryonic stem cell or a pluripotent cell; and judging whether or not the detection sequence is expressed in the embryonic stem cell or pluripotent cell after the introduction of the vector to select the embryonic stem cell or pluripotent cell judged to be one in which the expression exists as a gene modified embryonic stem cell or gene modified pluripotent cell. It is noted that the pluripotent cell is a cell having pluripotency and includes, for example, induced pluripotent stem cells (iPS cells).

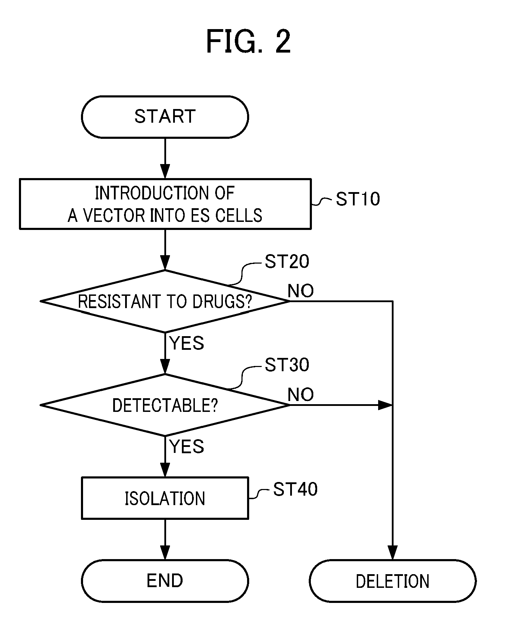

These procedures will be explained with reference to FIG. 2. The method shown in FIG. 2 corresponds to the case where a target is a gene which is sufficiently expressed in embryonic stem cells by using a vector having the sequence of a DNA fragment containing a regulatory sequence as shown in FIG. 1.

First, a vector is introduced into an embryonic stem cell (ST 10), and a gene modified embryonic stem cell is selected and isolated (ST40). Here, the selection of the gene modified embryonic stem cell can be made based on drug resistance (ST20) and, for example, on visual detection (ST30) because a detection marker and a resistance marker are expressed only when the DNA fragment is inserted into the target site. In this case, DNA is preferably extracted from the screened embryonic stem cells to confirm screening accuracy by southern analysis.

When a vector having a DNA fragment sequence containing a eukaryotic promoter sequence is used and a gene which is insufficiently expressed in an embryonic stem cell is a target, a gene modified embryonic stem cell is selected in the above ST40 as follows. Namely, because a detection marker and a resistance marker are expressed only when the DNA fragment is inserted into the genome of the embryonic stem cell, an embryonic stem cell with the DNA fragment inserted into its genome can be screened based on drug resistance (ST20) and visual detection (ST30). Next, the gene modified embryonic stem cell with the DNA fragment inserted into the target gene sequence is selected from the screened embryonic stem cell by a known method such as southern blotting. The above procedures are also applied to the creation of a gene modified pluripotent cell.

### Non-human knockout animal

The non-human knockout animal is provided with the above DNA fragment inserted into the genome. The knockout animals in the present invention are animals excluding humans, and usually include mammals such as mice, rats, rabbits, pigs, goats, and cows. These knockout animals are very useful for in-vivo analysis of the function of a knocked-out gene and for screening of a candidate compound of a therapeutic agent which will be explained later.

### [Method for manufacturing germline chimera animal]

A germline chimera animal is required to create these knockout animals. A method for manufacturing such an germline chimera animal involves the procedures comprising creating a chimera animal by using a gene modified embryonic stem cell or a gene modified pluripotent cell into which the above DNA fragment is introduced, and judging whether or not the detection sequence is expressed in the descendant of the chimera animal to select the chimera animal as a germline chimera animal when the expression is judged to exist in the descendant of the chimera animal.

Many detection sequences have already been expressed at birth though depending on the expression form of the target gene. For this, a phenotype associated with the expression of such a detection sequence is confirmed immediately after the birth. If the phenotype exists, it is found that a chimera mouse that is a parent of the descendant is a germline.

### [A method for manufacturing a non-human knockout animal]

Also, a method for manufacturing a non-human knockout animal involves the procedures comprising creating a chimera animal by using a gene modified embryonic stem cell or a gene modified pluripotent cell into which the above DNA fragment is introduced, and judging whether or not the detection sequence is expressed in the descendant of the chimera animal to select the chimera animal as a germline chimera animal when the expression is judged to exist in the descendant of the chimera animal; and creating a homozygote by using the germline chimera animal.

In the creation of the non-human knockout animal, for example, heterozygote mice obtained by crossing the above germline chimera animals with wild mice are made to cross with each other to obtain homozygote knockout mice.

Such a knockout animal is advantageous in that the trend of expression of a target gene can be grasped in vivo because the detection sequence is inserted into the target gene sequence.

### Screening method

A method for screening a candidate compound of a therapeutic agent for diseases comprises an administering procedure and a detecting procedure. Each procedure will be explained.

### (Administering procedure)

In the administering procedure, a specimen is administered to an animal in which the causal gene of the diseases is knocked out. The route of administration may be either oral or non-oral route. The site of administration may be whole body or local part of the body and may be properly determined taking an estimated administration form of a therapeutic agent into account. The oral administration includes sublingual administration, and examples of the route of non-oral administration include injections (for example, intravenous injection, subcutaneous injection, intramuscular injection, and drip) and percutaneous absorption.

### (Detecting procedure)

In the detecting procedure, after the administering procedure, it is detected whether or not the disease is improved in the non-human knockout animal. When an improvement in the disease is detected, it is suggested that the used specimen can complement or substitutionally perform the function of the causal gene of the diseases, the specimen may be judged to be a candidate compound of a therapeutic agent for diseases. The standard of the detection may be determined corresponding to the state of the improvement to be confirmed and may be any of a cell level, tissue level and individual level.

### Pharmaceutical composition

A pharmaceutical composition according to the present invention comprises an effective amount of a vector containing a DNA fragment and a complementary sequence of a partial sequence of a gene of which repressed expression is effective for the treatment or prevention of diseases. In this vector, the complementary sequence is located on both sides of the DNA fragment. Such a pharmaceutical composition ensures that homologous recombination between the target gene DNA and the DNA fragment occurs because the DNA fragment is sandwiched between the complementary sequences in the body of a subject to which the pharmaceutical composition is administered. Because the target gene is knocked out by this so that the expression thereof is repressed, diseases can be treated or prevented effectively.

The term "effective amount" in the description indicates the amount required to obtain desired treatment or preventive effect and is properly designed in accordance with the degree of the effect. Also, the route of administration of the pharmaceutical composition may be any of oral or non-oral administration and is properly designed corresponding to, for example, diseases to be treated or prevented.

In the case of oral administration, the pharmaceutical composition may contain additives which are generally used, such as a binder, inclusion agent, excipient, lubricating agent, disintegrator, and wetting agent, and may be made into preparations having various forms such as tablets, granules, fine grain agents, powders, and capsules. The pharmaceutical composition may be put into a liquid state such as an aqueous extract for internal-use, suspension, and emulsion syrup, or may be a state which is put into a dry state and redissolved prior to use.

In the case of non-oral administration, the pharmaceutical composition may contain additives such as a stabilizer, buffer, preservative, and isotonic agent, and is usually distributed in the condition that it is filled in a unit dose ample, or large dose container or tube. The pharmaceutical composition may be made into preparations having a powder form which can be redissolved in a proper carrier (for example, sterilized water) prior to use.

### EXAMPLES

### Example 1

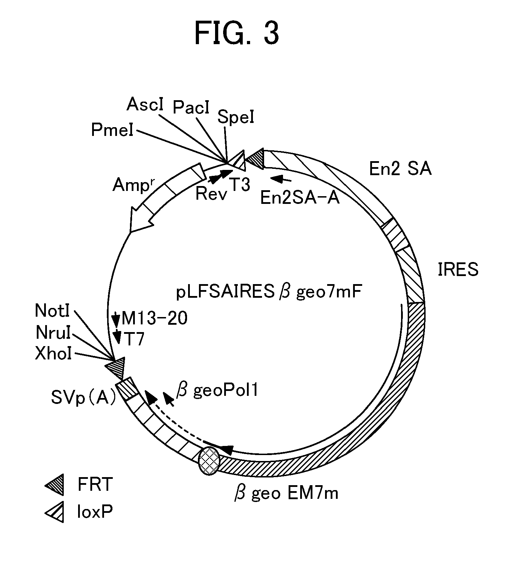

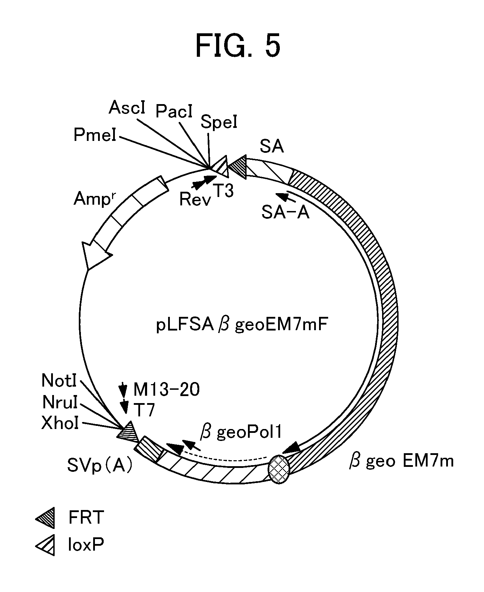

In this example, En2SA and IRES sequence were used as the regulatory sequence, a neomycin resistant gene sequence was used as the resistance sequence, a β-galactosidase gene was used as the detection sequence and an EM7m sequence obtained by transforming an EM7 promoter was used as the promoter sequence. This EM7m was located downstream of the detection sequence (see FIG. 3 and FIG. 5). As the backbone of a plasmid, "pBlueScriptII" (trademark, manufactured by STRATAGENE CORPORATION) (hereinafter referred to as "pBSK") was used. The cloning site of a vector, Pmel/AscI/PacI/SpeI shown in FIG. 3 was used as an adapter sequence to synthesize a DNA, and this synthesized DNA was inserted into the SacI/BamHl site of pBSK by a normal method. Similarly, a BamHI-loxP/FRT-SalI sequence was also produced by DNA synthesis and inserted into a BamHI/SalI site. The vector thus obtained was called pBSK-MCS-LF.

As the above En2SA, IRES sequence, β-galactosidase gene sequence and neomycin resistant gene sequence, the sequences inserted into the existing vector "pGT1. 81resβgeo" (Mountford P et al., PNAS, 91, 4303-4307, 1994) were used.

The promoter sequence EM7 which functioned in Escherichia coli was isolated from "pcDNA6/TR" (manufactured by Invitrogen Corporation) by PCR cloning. This product was inserted into pBSK to perform cloning (a vector obtained by this process is called "pBSK-EM7". When EM7 is inserted in-frame into the joint between the β-galactosidase gene sequence of "pGT1. 8Iresβgeo" and the neomycin resistant gene sequence, β-galactosidase and neomycin resistant protein are not expressed by a stop codon existing in the sequence. In light of this, "pBSK-EM7" was used to carry out the mutation of EM7 by PCR-mutagenesis to thereby obtain a mutant EM7 PCR product. Then, the sequence of this PCR product was confirmed.

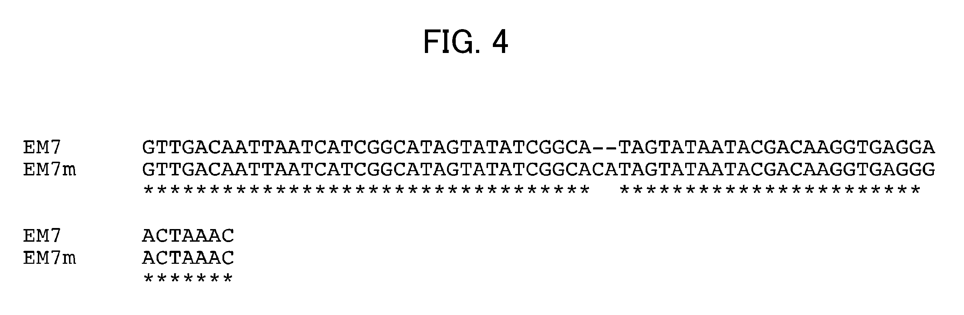

Based on this sequence, mutant EM7s which had no stop codon and could be inserted in-frame into the joint between the β-galactosidase and neomycin resistant gene sequences were screened and each mutant EM7 was inserted upstream of pBSK neomycin resistant gene sequence. The vector thus obtained was introduced into Escherichia coli to detect promoter activity based on whether or not a neomycin resistant gene was expressed. As a result, a mutant EM7 discriminated as one having excellent promoter activity is called a modified EM7, that is, "EM7m" (see FIG. 4). The base sequences of EM7 and EM7m are shown by SEQ ID NO: 1 and SEQ ID NO: 2 in the sequence listing.

Based on this information, EM7m having a PstI site on the 5' side and a sequence having the upstream of PstI sequence of the neomycin resistant gene sequence ORF on the 3' side were created by PCR and cloned into pBSK. This sequence was cut out of this pBSK by PstI. On the other hand, a Pstl-PstI region sequence located at the joint between the β-galactosidase gene sequence of pGT1. 8Iresβgeo and the neomycin resistant gene sequence was cut out and the upstream of a PstI sequence of the above EM7m-neomycin resistant gene sequence ORF was inserted instead. The vector thus obtained is called "pSAIRESβgeoEM7m".

The inserted sequence of pSAIRESβgeoEM7m was cut out by SalI. On the other hand, the above pBSK-MCS-LF was opened by SalI and SAIRESβgeoEM7m was inserted into this SalI site. The vector thus obtained was opened by XhoI and a SalI-NotI/NruI/Xhol-FRT-SalI sequence obtained by DNA synthesis was inserted. The strand bias of this sequence was confirmed by PCR, a restriction enzyme map and sequencing. This vector is referred to as "pLFSAIRESpgeoEM7mF" (FIG. 3).

### Example 2

In this example, an SA sequence was used as the regulatory sequence, a neomycin resistant gene sequence was used as the resistant sequence, a β-galactosidase gene sequence was used as the detection sequence and EM7m obtained in Example 1 was used as the promoter sequence (FIG. 5).

Many gene sequences used for the construction of the vector of FIG. 5 were fragments of pLFSAIRESβgeoEM7mF (FIG. 3) of Example 1. First, En2SA and IRES sequence which were the regulatory sequences were eliminated from pSAIRESβgeoEM7m of Example 1 by SalI/Hind III treatment.

The SA sequence was isolated from the existing vector "pSAneolox2βgal" (C.S. Raymond and P. Soriano, PLoS ONE, 2(1), e162, 2007) by PCR cloning. It is noted that in the PCR, a primer was used which was provided with a SalI site on the forward side and a HindIII site on the reverse side. This PCR product was inserted into pSAIRESβgeoEM7m from which En2SA and IRES sequence were eliminated and the accuracy of the sequence was confirmed by sequencing. The vector thus obtained is referred to as "pLFSAβgeoEM7mF" (FIG. 5).

### Example 3

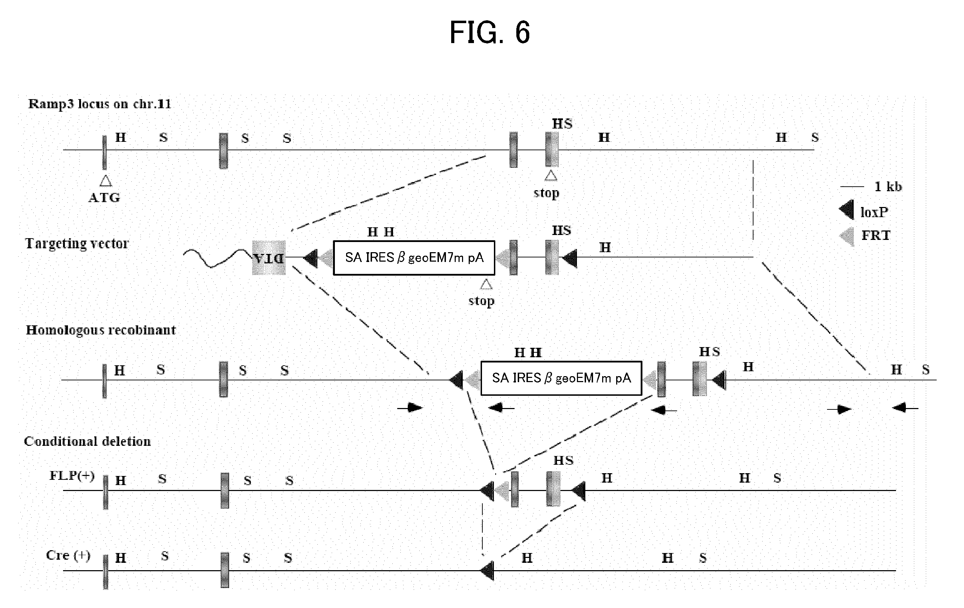

In this example, pSAIREβgeoEM7m was used to constitute a conditional targeting vector of an adrenomedullin receptor modifying factor "Receptor Activity Modifying Protein 3 (RAMP3) (see FIG. 6).

In the construction of this vector, the method according to P Liu et al., (Genome Research, 13, 476-484, 2003) was used as the fundamental techniques. The used Escherichia coli SW102 and SW106, and the loxP site insertion vector "pL452" were shared by Doctor N.G. Copeland in National Cancer Institute in USA. Also, a zeocin resistant gene substituted vector "p23loxZeo" and a recovery vector "pMCS-DTA" were shared by Doctor Yusa, in Osaka University in Japan. Also, a mouse BAC "RP23-69P8" including a RAMP3 sequence was procured from BACPAC Company. The sequence and information of the RAMP3 gene were obtained from NCBI.

First, an endogenous loxP site existing in RP23-69P8 was eliminated and RP23-69P8 from which the loxP site was eliminated was introduced into SW102 by the electroporation method (the vector thus obtained is referred to as "SW102+69P8"). Next, a zeocin resistant gene sequence fragment bilaterally having a sequence of the periphery of the RP23-69P8 endogenous loxP site was cut out from p23loxZeo by EcoRI treatment and purified. The purified fragment was introduced into SW102+69P8 by the electroporation method. This SW102+69P8 was kept at 42°C for 15 min. to thereby express an exo,bet,gam gene in SW102, thereby performing homologous recombination between the endogenous loxP region sequence and the zeocin resistant gene sequence. The transformed bacteria was screened by zeocin and chloramphenicol resistance, and further, a success in homologous recombination was confirmed by the PCR method (the vector thus obtained is referred to as "SW102+69P82eo").

The loxP sequence was introduced downstream of the fourth exon of RAMP3. The range extending to upstream and downstream for 400 bp centering around the RAMP3 gene region sequence into which the loxP site is introduced was isolated by PCR cloning. The accuracy of the sequence was confirmed by sequencing. Each of the PCR products was cloned into the BamHI/NotI and EcoRI/SalI sites of pL452, and a loxP-PGK promoter EM7Neo-loxP fragment flanked by the RAMP3 gene sequence fragment on the left and right sides thereof was cut out from the pL452 by NotI/SalI and purified. This purified fragment was introduced into SW102+69P8Zeo by the electroporation method. After that, this SW102+69P8Zeo was treated at 42°C for 15 min. to perform homologous recombination in the homologous regions of the introduced fragment and BAC in the SW102 bacteria. The transformed bacteria was screened by kanamycin resistance and a success in homologous recombination was confirmed by the PCR method (the vector thus obtained is referred to as "SW102+69P81oxPNeo2eo").

Next, BAC "69P8loxPNeoZeo was isolated from SW102+69P81oxPNeoZeo and reinserted into SW106 (the vector thus obtained is referred to as "SW106+69P8loxPNeo2eo"). This SWl06+69P8loxPNeoZeo was kept at 32°C for one hour in a 0.1% arabinose solution to thereby express a Cre gene of SW106 and to eliminate the PGK promoter EM7Neo between the loxP sequences. The transformed bacteria was screened based on chloramphenicol resistance and kanamycin sensitivity and the deficiency of PGK promoter EM7Neo was confirmed by the PCR method (the vector thus obtained is referred to as "SW106+69P8loxP2eo"). BAC was obtained by purification from this transformed bacteria and reinserted into SW102 (the vector thus obtained is referred to as "SW102+69P81oxP2eo").

A LFSAIRESβgeoEM7mF sequence was introduced upstream of the third exon of RAMP3. The range extending for 400 bp to the upstream and downstream centering around the sequence of the introduced part was isolated by PCR cloning. The accuracy of the sequence of the PCR product was confirmed by sequencing. Each of the PCR products was cloned into AscI/SpeI and Xhol/NotI sites of pLFSAIRESβgeoEM7mF. After that, a LFSAIRESβgeoEM7mF fragment flanked by the RAMP3 gene sequence fragment on the left and right sides thereof was cut out from pLFSAIRESβgeoEM7mF by AscI/NotI and purified. This purified fragment was introduced into SW102+69P81oxPZeo by the electroporation method.

The bacteria introduced into the vector was treated at 42°C for 15 minutes to perform homologous recombination in SW102, thereby inserting the LFSAIRESβgeoEM7mF sequence into the upstream of the third exon of RAMP3. The transformed bacteria was screened based on kanamycin and chloramphenicol resistance, and further, a success in homologous recombination was confirmed by the PCR method (the vector thus obtained is referred to as "SW102+69P8LFSAIRESβgeoEM7mFZeo").

Finally, the RMAP3 gene processed in the above manner was cut out of BAC "69P8LFSAIRESβgeoEM7mFZeo". A gene sequence 400 bp existing in the range extending for 2 kb to the upstream of the first exon of the RAMP3 gene region sequence and for 6 kb to the downstream of the third exon of the RAMP3 gene region sequence was isolated by PCR cloning. The accuracy of these sequences was confirmed by sequencing. Each of the PCR products was cloned into XhoI/XbaI and XbaI/KpnI sites of pMCS-DTA. After that, the pMCS-DTA having the RMAP3 gene sequence was purified and linearized by XbaI treatment. This DNA fragment was introduced into the above SW102+69P8LFSAIRESβgeoEM7mFZeo by the electroporation method. The cell into which the vector was introduced was treated at 42°C for 15 min. to perform homologous recombination between BAC and the introduced DNA fragment in SW102. SW102 bearing pMCS-DTA into which the whole region of the RAMP3 gene sequence processed for conditional knockout use by homologous recombination between the RAMP3 gene sequences of pMCS-DTA was inserted was screened by kanamycin and ampicilline resistances. Moreover, a success in homologous recombination was confirmed by the PCR method and a restriction enzyme map. A plasmid was obtained from this transformed bacteria by purification. This plasmid is referred to as a RAMP3 Conditional Gene Targeting Vector "pRSCKO1".

### Example 4

In this example, the above pR3CKO1 was used to obtain a RAMP3 gene transformed ES cell. The technique described in T Yagi et al., (Anal. Biochem, 214, 70-76, 1993) was adopted as a basic method. The above pR3CKO1 was linearized by a restriction enzyme PmeI to create a conditional gene targeting vector for introducing ES cells use. As the ES cell, "E14.1" (R. Kuhn et al., Science, 254, 707-710, 1991) was used.

The ES cell was proliferated by culturing, and suspended in a concentration of 2.5x10⁷ cells/ml just before use. This suspension was cooled on ice until R3CKO1 was introduced. The introduction of R3CKO1 was accomplished by electroporation using "GenePulser" (manufactured by BIORAD Corporation). 0.75 mL of an ES cell solution (1.1x10⁷ cells) and 50 uL of an adjusted R3CKO1 DNA (50 ug) solution were carefully added in a cooling cuvet 0.4 cm in width by a 1 mL chip. An electric pulse was applied once in the condition set to 230 V and 500 uF. After allowed to stationarily stand at room temperature for 10 min., the ES cells in the cuvet were suspended in a 24 mL medium (ESM) for ES cells and the suspension medium was planted in four culturing dishes 10 cm in diameter in an amount of 4.5 mL per dish. In these culturing dishes, primary fibroblasts isolated from embryonic day 12.5 to 14.5 which had a neomycin resistant sequence were planted as feeder cells in advance. Medium exchange was made after 24 hrs. and two of each culturing dish were exchanged for an ESM medium to which 140 µg/mL of Geneticin (trademark) was added to start screening of drugs. Thereafter, the ESM medium to which Geneticin was added was exchanged for a new one every day.

49 neomycin resistant ES cell colonies were isolated 8 days after the start of the screening of drugs, and each colony was separated into single cell. These single cells were each divided into two: one being planted in a 24-hole culture well coated with gelatin and the other being planted in a 24-hole culture well in which neomycin resistant feeder cells were planted, followed by successive culturing.

When the cells were sufficiently proliferated, high-molecular DNAs were isolated from the former by a normal method, and the obtained DNA was used as a preliminary sample for PCR or southern blot used in the subsequent processes.

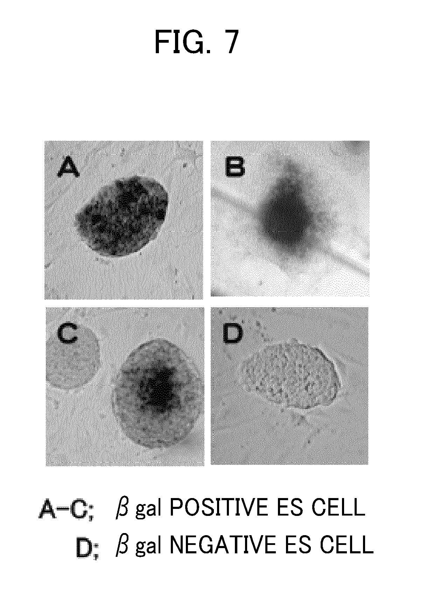

The latter was subjected to medium exchange the next day and then, cells were frozen when ES cell colonies were increased. At this time, a part of the ES cell suspension solution was planted in a 24-hole or 96-hole culture well in which feeder cells were planted. When ES cells were increased in each well, β-galactosidase dyeing was performed. As a result, ES cells in 11 wells were positive (see FIG. 7). With regard to this β-galactosidase positive ES cell group, homologous recombination was confirmed by PCR specific to the 5'-side and 3'-side of the RAMP3 gene sequence, and as a result, two populations of ES cells were confirmed to be RAMP3 gene modified ES cells.

## Claims

1. A DNA fragment comprising a detection sequence that codes for a detection marker other than a drug resistance marker, a resistance marker that codes for a resistance marker for a drug that inhibits the proliferation of one or more species of at least prokaryotic organisms, a prokaryotic promoter sequence that is located upstream of the resistance sequence and functions in the prokaryotic organisms, and a regulatory sequence that is located the most upstream of the sequences and induces the expression of the sequences located downstream thereof only when it is inserted into a target region of the genome of at least one species of eukaryotic organism, wherein;
the detection sequence and the resistance sequence are operably arranged.

2. The DNA fragment according to Claim 1, wherein the regulatory sequence is a sequence constituting one or more types selected from the group consisting of a sequence internal ribosome entry site and a splicing acceptor.

3. A DNA fragment comprising a detection sequence that codes for a detection marker which is visually detectable, a resistance sequence that codes for a resistance marker for a drug that inhibits the proliferation of one or more species of at least prokaryotic organisms, a prokaryotic promoter sequence that is located upstream of the resistance sequence and functions in the prokaryotic organisms, and a eukaryotic promoter sequence that is located the most upstream of the sequences and constitutively functions by at least one species of eukaryotic organisms, wherein;
the detection sequence and the resistance sequence are operably arranged.

4. The DNA fragment according to any one of Claims 1 to 3, wherein the detection marker is a visually detectable one.

5. The DNA fragment according to any one of Claims 1 to 4, wherein the resistance marker further imparts resistance to drugs that inhibit the proliferation of eukaryotic organisms.

6. A vector comprising the sequence of the DNA fragment claimed in any one of Claims 1 to 5.

7. The vector according to Claim 6, the vector further comprising a complementary sequence of a targeting sequence constituting part of an animal genome.

8. A host cell comprising the vector claimed in Claim 6 or 7 introduced thereinto.

9. A method for manufacturing a gene modified embryonic stem cell or a gene modified pluripotent cell, the method comprising:
introducing the vector claimed in Claim 7 into an embryonic stem cell or a pluripotent cell; and
judging whether or not the detection sequence is expressed in the embryonic stem cell or pluripotent cell after the introduction of the vector to select the embryonic stem cell or pluripotent cell judged to be one in which the expression exists as a gene modified embryonic stem cell or gene modified pluripotent cell.

10. A method for manufacturing an germline chimera animal, the method comprising:
creating a chimera animal by using a gene modified embryonic stem cell or a gene modified pluripotent cell into which the DNA fragment claimed in any one of Claims 1 to 5 is introduced; and
judging whether or not the detection sequence is expressed in the descendant of the chimera animal to select the chimera animal as a germline chimera animal when the expression is judged to exist in the descendant of the chimera animal.

11. A method for manufacturing a non-human knockout animal, the method comprising:
introducing the vector claimed in Claim 7 into an embryonic stem cell or a pluripotent cell;
judging whether or not the detection sequence is expressed in the embryonic stem cell or pluripotent cell after the introduction of the vector to select the embryonic stem cell or pluripotent cell judged to be one in which the expression exists as a gene modified embryonic stem cell or gene modified pluripotent cell;
creating a germline chimera animal by using the gene modified embryonic stem cell or gene modified pluripotent cell; and
creating a homozygote by using the germline chimera animal.

12. A method for manufacturing a non-human knockout animal, the method comprising:
creating a chimera animal by using a gene modified embryonic stem cell or a gene modified pluripotent cell into which the DNA fragment claimed in any one of Claims 1 to 5 is introduced; and
judging whether or not the detection sequence is expressed in the descendant of the chimera animal to select the chimera animal as a germline chimera animal when the expression is judged to exist in the descendant of the chimera animal; and
creating a homozygote by using the germline chimera animal.

13. A method for screening a candidate compound of a therapeutic agent for diseases, the method comprising:
administering a specimen to a non-human knockout animal in which the causal gene of the diseases is knocked out by introducing the vector claimed in Claim 7; and
detecting whether or not the disease is improved in the non-human knockout animal.

14. A pharmaceutical composition comprising an effective amount of a vector containing the DNA fragment claimed in any one of Claims 1 to 5 and a complementary sequence of a sequence constituting a gene of which repressed expression is effective for the treatment or prevention of diseases, the complementary sequence being located on both sides of the DNA fragment.


SEQUENCE LISTING

<110>  Immuno-Biological Laboratories Co., Ltd.

<120>  DNA fragment and therapeutic composition

<130>  ATF-042PCT

<150>  JP 2009-167580
<151>  2009-07-16

<160>  2

<170>  PatentIn version 3.1

<210>  1
<211>  65
<212>  DNA
<213>  Escherichia coli

<400>  1
gttgacaatt aatcatcggc atagtatatc ggcatagtat aatacgacaa ggtgaggaac     60

taaac                                                                 65


<210>  2
<211>  67
<212>  DNA
<213>  Artificial
<220>
<223>  The nucleotide comprising this sequence has a promotor activity.

<400>  2
gttgacaatt aatcatcggc atagtatatc ggcacatagt ataatacgac aaggtgaggg     60

actaaac                                                               67